(19) Europäisches Patentamt

European Patent Office

Office européen des brevets

(11) Numéro de publication: **0 513 323 B1**

(12) **FASCICULE DE BREVET EUROPEEN**

(45) Date de publication de fascicule du brevet: **01.02.95** (51) Int. Cl.⁶: **C07C 2/32**, C08F 4/642, C07C 11/02

(21) Numéro de dépôt: **92901487.6**

(22) Date de dépôt: **27.11.91**

(86) Numéro de dépôt internationale : **PCT/FR91/00945**

(87) Numéro de publication internationale : **WO 92/10446 (25.06.92 92/14)**

(54) **PROCEDE DE CONVERSION DE L'ETHYLENE EN OLEFINES ALPHA LEGERES.**

(30) Priorité: **04.12.90 FR 9015268**

(43) Date de publication de la demande: **19.11.92 Bulletin 92/47**

(45) Mention de la délivrance du brevet: **01.02.95 Bulletin 95/05**

(84) Etats contractants désignés: **DE FR GB IT NL**

(56) Documents cités:
**EP-A- 0 114 416
DE-A- 2 318 953
FR-A- 2 243 173
US-A- 3 206 523
US-A- 3 879 485**

(73) Titulaire: **INSTITUT FRANCAIS DU PETROLE
4, avenue de Bois Préau
F-92502 Rueil-Malmaison (FR)**

(72) Inventeur: **CHAUVIN, Yves
64, avenue du Général-Leclerc
F-78230 Le Pecq (FR)**

Inventeur: **COMMEREUC, Dominique
32, rue Abel-Vacher
F-91190 Meudon (FR)**
Inventeur: **FORESTIERE, Alain
1369, chemin du Pelet
F-69390 Vernaison (FR)**
Inventeur: **HUGUES, François
10, chemin du Clos-Chaland,
Charly
F-69390 Vernaison (FR)**
Inventeur: **SAUSSINE, Lucien
2, place des Frères-Tissandier
F-78290 Croissy-sur-Seine (FR)**

(74) Mandataire: **Andreeff, François et al
INSTITUT FRANCAIS DU PETROLE
4, avenue de Bois-Préau
F-92506 Rueil-Malmaison Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3. 10/3.09/3.3.3)

## Description

La présente invention concerne un procédé d'oligomérisation de l'éthylène en oléfines alpha légères, principalement en butène-1, hexène-1 et octène-1.

Dans le brevet US 2 943 125, K. Ziegler a décrit une méthode de dimérisation de l'éthylène en butène-1 au moyen d'un catalyseur obtenu par le mélange de trialkylaluminium et d'un tétraalcoolate de titane ou de zirconium ; lors de la réaction se forme également une certaine quantité de polyéthylène de haute masse moléculaire qui gêne considérablement la mise en oeuvre. Plusieurs améliorations ont été proposées pour diminuer le taux de polymère, en particulier dans le brevet US 3 686 350 qui préconise l'emploi de composés organiques du phosphore, conjointement avec les éléments du catalyseur, dans le brevet US 4 101 600 qui décrit le traitement du catalyseur par de l'hydrogène ou dans le brevet US 3 879 485 qui revendique l'utilisation de divers éthers comme solvants du milieu réactionnel.

L'oligomérisation de l'éthylène en oléfines alpha de poids moléculaires variés est connue depuis les années 1960-1965. A côté de méthodes mettant en jeu une réaction de croissance de chaîne stoechiométrique, par exemple à partir d'un composé organoaluminique, il a été trouvé plusieurs méthodes catalytiques faisant appel à des métaux divers tels que le titane, le zirconium, le chrome, le nickel ou les terres rares, mis en jeu le plus souvent dans des recettes de type Ziegler. Toutes ces méthodes fournissent des mélanges d'oligomères ayant un nombre de carbone qui peut varier largement et qui est compris entre 4 et 30 ou même supérieur à 30. Ces mélanges conviennent bien pour les utilisations jusque là dévolues à ces oligomères (plastifiants, détergents notamment).

Depuis quelques années est apparue une demande croissante en oligomères inférieurs, essentiellement butène-1, hexène-1 et octène-1, qui trouvent leur utilisation comme comonomères avec l'éthylène dans la fabrication du polyéthylène basse densité linéaire.

Il a été maintenant trouvé selon la présente invention que les catalyseurs obtenus en mélangeant au moins un composé particulier d'aluminium avec au moins un mélange préformé d'au moins un éther et d'au moins un zirconate d'alkyle présentent une sélectivité inattendue pour la formation des oligomères inférieurs, principalement le butène-1, l'hexène-1 et l'octène-1.

L'invention concerne ainsi un procédé amélioré de conversion de l'éthylène en oléfines alpha légères dans lequel l'éthylène est mis en contact avec au moins un catalyseur obtenu par réaction d'un mélange préformé de zirconate d'alkyle et d'éther, dans un rapport molaire éther/zirconate de 0,5:1 à 10:1, avec au moins un composé d'aluminium de formule générale $AlR_nX_{3-n}$ dans laquelle R est un radical hydrocarbyl, X est un atome de chlore ou de brome et n est un nombre compris entre 1 et 2.

Les éthers sont ainsi utilisés dans un rapport molaire de 0,5 à 10, de préférence de 1 à 4, plus particulièrement de 2 à 3 moles d'éther par mole de zirconate d'alkyle. Sans être lié par aucune théorie, on peut penser que l'éther se complexe sur l'atome de zirconium permettant l'hexacoordination que le zirconium ne réalise autrement qu'en s'autoassociant.

Les éthers utilisés dans l'invention peuvent être des monoéthers ou des polyéthers. On utilise de préférence les monoéthers, comme par exemple le diéthyléther, le diisoamyléther, le méthyltertiobutyléther, le tétrahydrofuranne.

Les zirconates d'alkyle utilisés dans l'invention répondent habituellement à la formule générale $Zr(OR')_4$ dans laquelle R' est un radical alkyle, linéaire ou ramifié, comportant de préférence de 2 à 8 atomes de carbone. On peut utiliser par exemple le zirconate de tétraéthyle, le zirconate de tétra-n-propyle, le zirconate de tétra-n-butyle, le zirconate de tétra-2-éthylhexyle.

Les composés de l'aluminium utilisés dans l'invention sont représentés par la formule générale $AlR_nX_{3-n}$ dans laquelle R est un radical hydrocarbyl, de préférence alkyle comprenant de 2 à 6 atomes de carbone, X est un atome de chlore ou de brome, de préférence un atome de chlore et n est un nombre compris entre 1 et 2 (n peut être égal à 1 ou à 2 notamment). On peut citer à titre d'exemples le chlorodiéthylaluminium, le dichloroéthylaluminium, le sesquichlorure d'éthylaluminium ou leurs mélanges.

Les composants du catalyseur peuvent être mis en contact au sein d'un hydrocarbure, par exemple un hydrocarbure saturé comme l'hexane ou l'heptane, et/ou d'un ou des sous-produits d'oligomérisation tels que les décènes, dodécènes ou les oligomères supérieurs.

Le rapport molaire entre le composé d'aluminium et le zirconate d'alkyle est d'environ 1:1 à 30:1, de préférence d'environ 5:1 à 20:1. La concentration de zirconium dans la solution catalytique ainsi préparée est avantageusement comprise entre $10^{-4}$ et 0,5 mole par litre et de préférence entre $2.10^{-3}$ et 0,1 mole par litre. La température à laquelle se fait la réaction du mélange préformé de zirconate d'alkyle et d'éther avec le composé d'aluminium est habituellement comprise entre -10 et +50 °C, de préférence entre 0 et +40 °C et par exemple égale à la température ambiante (15 à 25 °C). Cette réaction peut être effectuée sous une atmosphère de gaz inerte ou d'éthylène.

La solution catalytique ainsi obtenue peut être utilisée telle quelle ou elle peut être diluée par addition des produits de la réaction.

Dans un mode particulier de mise en oeuvre de la réaction catalytique d'oligomérisation en discontinu, on introduit un volume choisi de la solution catalytique, préparée comme décrit ci-dessus, dans un réacteur muni des habituels systèmes d'agitation et de refroidissement, puis on pressurise par de l'éthylène à une pression généralement comprise entre 0,5 et 10 MPa, de préférence entre 1 et 6 MPa ; on maintient la température en général entre 20 et 80 °C, de préférence entre 40 et 75 °C. On alimente le réacteur d'oligomérisation par de l'éthylène à pression constante jusqu'à ce que le volume total de liquide produit représente entre 2 et 50 fois le volume de la solution catalytique primitivement introduit. On détruit alors le catalyseur, par exemple par addition d'eau, et on soutire et sépare les produits de la réaction et le solvant éventuel.

En cas d'opération continue, la mise en oeuvre est de préférence la suivante : la solution catalytique est injectée en même temps que l'éthylène dans un réacteur agité par les moyens mécaniques classiques ou par une recirculation extérieure. La température est maintenue entre 20 et 80 °C, de préférence entre 40 et 75 °C, et la pression doit être suffisante pour que tous les composés se trouvent en phase liquide ; selon les débits de catalyseur et d'éthylène, cette pression sera comprise entre 0,5 et 10 MPa. Par une vanne de détente, qui maintient la pression constante, s'écoule une partie du mélange réactionnel, à un débit massique égal au débit massique des fluides introduits. Le fluide ainsi détendu est envoyé dans un système de colonnes à distiller qui permet de séparer les oligomères de l'éthylène d'une part, éthylène qui peut être renvoyé au réacteur, puis les oligomères entre eux d'autre part. Les produits lourds contenant le catalyseur peuvent être incinérés.

Les exemples suivants illustrent l'invention.

## EXEMPLE 1

Dans un autoclave en acier inoxydable d'un volume de 250 ml, muni d'une double enveloppe permettant de réguler la température par circulation d'eau, ou introduit dans l'ordre, sous atmosphère d'argon et à la température ambiante : 0,79 mmole (c'est-à-dire $0,79.10^{-3}$ mole) de zirconate de tétra-n-butyle $Zr(OBu)_4$ et 1,58 mmole de tétrahydrofuranne en solution dans 50 ml de n-heptane, puis 4,40 mmoles de sesquichlorure d'éthylaluminium également en solution dans 50 ml de n-heptane.

Après quelques minutes de réaction, la température est portée à 60 °C tout en introduisant dans l'autoclave de l'éthylène à une pression constante de 4 MPa (la pression est maintenue à cette valeur par introduction complémentaire d'éthylène au fur et à mesure qu'il est consommé).

Après 60 minutes de réaction, l'introduction d'éthylène est arrêtée et le catalyseur est détruit par injection sous pression de 2 ml d'eau ; on a consommé au total 70 g d'éthylène.

La composition des produits obtenus est donnée dans le tableau 1. On recueille en outre 0,55 % en poids de polymère solide par rapport à l'éthylène consommé.

## EXEMPLE 2

Dans le même appareillage que celui qui a été utilisé dans l'exemple 1 et dans les mêmes conditions, à ceci prés qu'on a introduit deux fois plus de tétrahydrofuranne, on a consommé 35,7 g d'éthylène pendant la réaction que l'on a arrêtée après 170 minutes.

La composition des produits obtenus est donnée dans le tableau 1. On a recueilli en outre 3,8 % en poids de polymère solide par rapport à l'éthylène consommé.

## EXEMPLE 3

Dans le même appareillage que celui qui a été utilisé dans l'exemple 1, on introduit dans l'ordre, sous atmosphère d'argon et à la température ambiante : 0,19 mmole de $Zr(OBu)_4$ et 0,39 mmole de tétrahydrofuranne en solution dans 10 ml de n-heptane, puis 1,06 mmole de sesquichlorure d'éthylaluminium en solution dans 15 ml de n-heptane.

Après quelques minutes de réaction, la température est portée à 70 °C tout en introduisant dans l'autoclave de l'éthylène à une pression constante de 4MPa.

Après 180 minutes de réaction l'introduction d'éthylène est arrêtée et le catalyseur est détruit par injection sous pression de 2ml d'eau ; on a consommé au total 61,6 g d'éthylène.

La composition des produits obtenus est donnée dans le tableau 1. On a recueilli en outre 1,2 % en poids de polymère solide par rapport à l'éthylène consommé.

EXEMPLE 4

Dans le même appareillage que celui qui a été utilisé dans l'exemple 1, on introduit dans l'ordre, sous atmosphère d'éthylène et à la température ambiante : 0,39 mmole de $Zr(OBu)_4$ et 0,79 mmole de tétrahydrofuranne en solution dans 50 ml de n-heptane, puis 2,17 mmoles de sesquichlorure d'éthylaluminium également en solution dans 50 ml de n-heptane.

Après quelques minutes de réaction, la température est portée à 75 °C et la pression d'éthylène est ajustée à 4MPa.

Après 55 minutes de réaction, l'introduction d'éthylène est arrêtée et le catalyseur est détruit par injection sous pression de 2 ml d'eau ; on a consommé au total 77,0 g d'éthylène en 55 minutes.

La composition des produits obtenus est donnée dans le tableau 1. On recueille en outre 0,69 % en poids de polymère solide par rapport à l'éthylène consommé.

EXEMPLE 5 (comparatif)

Dans le même appareillage que celui qui a été utilisé dans l'exemple 4 et dans les mêmes conditions, à ceci près qu'on n'introduit pas de tétrahydrofuranne, on a consommé 71,6 g d'éthylène en 100 minutes de réaction.

La composition des produits obtenus est donnée dans le tableau 1. On a recueilli en outre 4,6 % en poids de polymère solide par rapport à l'éthylène consommé.

Cet exemple montre bien que la technique antérieure qui y est utilisée donne des résultats moins satisfaisants que ceux obtenus dans les exemples selon l'invention et notamment une répartition de produits défavorable pour les oléfines alpha légères $C_4$, $C_6$ et $C_8$.

EXEMPLE 6

Dans le même appareillage que celui qui a été utilisé dans l'exemple 1, on introduit dans l'ordre, sous atmosphère d'argon et à la température ambiante : 0,38 mmole de $Zr(OBu)_4$ et 0,76 mmole de tétrahydrofuranne en solution dans 10 ml de n-heptane, puis un mélange de 1,14 mmole de $AlEt_3$ et de 3,42 mmoles de AlEtCl2 (ce mélange équivaut à 2,28 mmole de sesquichlorure d'éthylaluminium) en solution dans 15 ml de n-heptane.

Après quelques minutes de réaction, la température est portée à 60 °C tout en introduisant dans l'autoclave de l'éthylène à une pression constante de 4 MPa.

Après 270 minutes de réaction, l'introduction d'éthylène est arrêtée et le catalyseur est détruit par injection sous pression de 2 ml d'eau ; on a consommé au total 106 g d'éthylène.

La composition des produits obtenus est donnée dans le tableau 1. On a recueilli en outre 1,2 % en poids de polymère solide par rapport à l'éthylène consommé.

EXEMPLE 7

Dans un autoclave en acier inoxydable d'un volume de 1 litre, muni d'une double enveloppe permettant de réguler la température par circulation d'eau, on introduit dans l'ordre, sous atmosphère d'azote et à la température ambiante : 0,78 mmole de $Zr(OBu)_4$ et 1,53 mmole de tétrahydrofuranne en solution dans 100 ml de n-heptane, puis 4,68 mmoles de sesquichlorure d'éthylaluminium également en solution dans 100 ml de n-heptane.

Après quelques minutes de réaction, la température est portée à 50 °C tout en introduisant dans l'autoclave de l'éthylène à une pression constante de 4 MPa.

Après 210 minutes de réaction, l'introduction d'éthylène est arrêtée et le catalyseur est détruit par injection sous pression de 5 ml d'eau ; on a consommé au total 195 g d'éthylène.

La composition des produits obtenus est donnée dans le tableau 1. On recueille en outre 0,31 % en poids de polymère solide par rapport à l'éthylène consommé.

TABLEAU I

|  | Répartition des produits obtenus (% poids) | | | | Teneur en oléfines alpha obtenues (% poids) | | |
|---|---|---|---|---|---|---|---|
| Exemple | $C_4$ | $C_6$ | $C_8$ | $C_{10}^+$ | dans $C_4$ | dans $C_6$ | dans $C_8$ |
| 1 | 30,0 | 27,0 | 15,9 | 27,1 | 97,1 | 91,0 | 91,2 |
| 2 | 42,4 | 22,8 | 9,9 | 24,9 | 97,1 | 92,1 | - |
| 3 | 30,6 | 32,6 | 19,2 | 17,6 | 98,2 | 87,3 | 92,2 |
| 4 | 26,1 | 27,7 | 18,7 | 27,5 | 97,7 | 90,6 | 95,4 |
| 5 | 16,9 | 17,7 | 28,3 | 37,1 | 94,4 | 83,0 | - |
| 6 | 38,1 | 35,4 | 17,4 | 9,1 | 96,0 | 91,9 | 92,1 |
| 7 | 32,6 | 27,9 | 18,0 | 21,5 | 97,0 | 95,0 | - |

(avec $C_4$ = butènes, $C_6$ = hexènes, $C_8$ = octènes, $C_{10}^+$ = décènes et oléfines supérieures).

## Revendications

1. Procédé de conversion de l'éthylène en oléfines alpha légères dans lequel l'éthylène est mis en contact avec au moins un catalyseur obtenu par réaction d'un mélange préformé de zirconate d'alkyle et d'éther, dans un rapport molaire éther/zirconate de 0,5:1 à 10:1, avec au moins un composé d'aluminium de formule générale $AlR_nX_{3-n}$ dans laquelle R est un radical hydrocarbyl, X est un atome de chlore ou de brome et n est un nombre compris entre 1 et 2.

2. Procédé selon la revendication 1 dans lequel ledit rapport molaire éther/zirconate est compris entre 1:1 et 4:1.

3. Procédé selon l'une des revendications 1 et 2 dans lequel ladite réaction est effectuée dans un hydrocarbure.

4. Procédé selon l'une des revendications 1 à 3 dans lequel ledit éther est le diéthyléther, le diisoamyléther, le méthyltertiobutyléther ou le tétrahydrofuranne.

5. Procédé selon la revendication 4 dans lequel ledit éther est le tétrahydrofuranne.

6. Procédé selon l'une des revendications 1 à 5 dans lequel ledit zirconate d'alkyle est le zirconate de tétra-n-propyle ou le zirconate de tétra-n-butyle.

7. Procédé selon l'une des revendications 1 à 6 dans lequel ledit composé d'aluminium est le sesquichlorure d'éthylaluminium.

8. Procédé selon l'une des revendications 1 à 7 dans lequel le rapport molaire entre le composé d'aluminium et le zirconate d'alkyle est d'environ 1:1 à 30:1 et la concentration en zirconium est comprise entre $10^{-4}$ et 0,5 mole par litre.

9. Procédé selon l'une des revendications 1 à 8 dans lequel ladite réaction s'effectue à une température comprise entre 0 et +40 °C et sous une atmosphère de gaz inerte ou d'éthylène.

10. Procédé selon l'une des revendications 1 à 9 dans lequel la conversion de l'éthylène en oléfines alpha légères s'effectue à une température comprise entre +40 et +75 °C et sous une pression comprise entre 1 et 6 MPa.

## Claims

1. Process for the conversion of ethylene into light alpha olefins, wherein the ethylene is contacted with at least one catalyst obtained by reacting a performed mixture of alkyl zirconate and ether in an ether zirconate molar ratio of 0.5 : 1 to 10 : 1, with at least one aluminum compound of general formula $AlR_nX_{3-n}$, in which R is a hydrocarbyl radical, X is a chlorine or bromine atom and n is a number between 1 and 2.

2. Process according to claim 1, wherein said ether/zirconate molar ratio is between 1:1 and 4:1.

3. Process according to either of the claims 1 and 2, wherein the reaction is performed in a hydrocarbon.

4. Process according to any one of the claims 1 to 3, wherein the ether is diethyl ether, diisoamyl ether, methyl tert. butyl ether or tetrahydrofuran.

5. Process according to claim 4, wherein the ether is tetrahydrofuran.

6. Process according to any one of the claims 1 to 5, wherein the alkyl zirconate is tetra-n-propyl zirconate or tetra-n-butyl zirconate

7. Process according to any one of the claims 1 to 6, wherein the aluminium compound is ethyl aluminium sesquichloride.

8. Process according to any one of the claims 1 to 7, wherein the molar ratio between the aluminium compound and the alkyl zirconate is approximately 1:1 to 30:1 and the zirconium concentration is between $10^{-4}$ and 0.5 mole per litre.

9. Process according to any one of the claims 1 to 8, wherein the reaction takes place at a temperature between 0 and $+40°C$ and under an ethylene or inert gas atmosphere.

10. Process according to any one of the claims 1 to 9, wherein the conversion of ethylene into light alpha olefins takes place at a temperature between $+40$ and $+75°C$ and under a pressure between $+1$ and 6 MPa.

**Patentansprüche**

1. Verfahren zur Umwandlung von Ethylen in leichte alpha-Olefine, worin Ethylen mit wenigstens einem Katalysator in Kontakt gebracht wird, der durch Umsetzung einer vorgefertigten Mischung aus Alkylzirkonat und Ether, in einem Ether/Zirkonat - Molverhältnis von 0,5:1 bis 10:1, mit wenigstens einer Aluminiumverbindung der allgemeinen Formel $AlR_nX_{3-n}$, in welcher R einen Kohlenwasserstoffrest bedeutet, X ein Chlor- oder Bromatom und n eine Zahl zwischen 1 und 2 ist, erhalten wird.

2. Verfahren nach Anspruch 1, worin das Ether/Zirkonat - Molverhältnis zwischen 1:1 und 4:1 liegt.

3. Verfahren nach einem der Ansprüche 1 und 2, worin die Umsetzung in einem Kohlenwasserstoff bewirkt wird.

4. Verfahren nach einem der Ansprüche 1 bis 3, worin der Ether Diethylether, Diisoamylether, Methyl-t-butylether oder Tetrahydrofuran ist.

5. Verfahren nach einem der Ansprüche 1 bis 4, worin der Ether Tetrahydrofuran ist.

6. Verfahren nach einem der Ansprüche 1 bis 5, worin das Alkylzirkonat Tetra-n-propylzirkonat oder Tetra-n-butylzirkonat ist.

7. Verfahren nach einem der Ansprüche 1 bis 6, worin die Aluminiumverbindung Ethylaluminiumsesquichlorid ist.

8. Verfahren nach einem der Ansprüche 1 bis 7, worin das Molverhältnis zwischen der Aluminiumverbindung und dem Alkylzirkonat etwa 1:1 bis 30:1 und die Zirkoniumkonzentration zwischen $10^{-4}$ und 0,5 mol/l beträgt.

9. Verfahren nach einem der Ansprüche 1 bis 8, worin die Umsetzung bei einer Temperatur von 0 bis $+40°C$ und unter einer Atmosphäre von Inertgas oder Ethylen stattfindet.

**10.** Verfahren nach einem der Ansprüche 1 bis 9, worin die Umwandlung von Ethylen in leichte alpha-Olefine bei einer Temperatur zwischen +40°C und +75°C und unter einem Druck zwischen 1 und 6 MPa stattfindet.